# EUROPEAN PATENT APPLICATION

(11) **EP 4 796 118 A2**
(43) Date of publication of application: **26.08.2026**
(21) Application number: 26158899.0
(22) Date of filing: 16.02.2026
(51) Int. Cl.: A61K 35/30, A61P 35/00, C12N 5/00, A61P 37/00

(54) **EXOSOME OBTAINED VIA CORNEAL TISSUE, MANUFACTURING METHOD, USE AND PHARMACEUTICAL COMPOSITION THEREOF**

(30) Priority: 21.02.2025 US 202563761444 P
(71) Applicant: Shine-On BioMedical Co., Ltd., 403020 Taichung City (TW); China Medical University, Taichung City 406040 (TW); China Medical University Hospital, Taichung City 404 (TW)
(72) Inventor: Ho, Hui-Chun, 116056 Taipei City (TW); Tsai, Yi-Yu, 403410 Taichung City (TW); Cho, Der-Yang, 404327 Taichung City (TW); Lai, Hung-Yin, 802574 Kaohsiung City (TW); Wu, Hao-Hsiang, 328006 Taoyuan City (TW); Chiang, Chun-Chi, 408007 Taichung City (TW); Chiang, Hung-Che, 403020 Taichung City (TW); Lee, Oscar Kuang-Sheng, 111050 Taipei City (TW); Yu, Ni-Yen, 403020 Taichung City (TW)
(74) Representative: Cabinet Chaillot

(57) **Abstract**

A corneal tissue exosome collected after rapid culture of a corneoscleral tissue directly following corneal transplantation is disclosed. The overall manufacturing process is simple, eliminating the procedures of thawing, isolation, and cell culture in conventional cell culture processes, and possesses high production yield and homogeneity, while reducing the generation of biomedical waste. The corneal tissue exosome obtained from the corneoscleral tissue exhibits specific protein expression and possesses potential for treating wound repair, ocular diseases, cancer, and immune-related diseases.

## Description

### FIELD OF INVENTION

The present invention relates to an exosome and, more particularly, to a corneal tissue exosome obtained by culturing a corneoscleral tissue, a manufacturing method thereof, a use thereof, and a pharmaceutical composition comprising the same.

### BACKGROUND OF THE INVENTION

In the prior art, techniques have been developed to employ exosomes as carriers for encapsulating drugs, proteins, or nucleic acids to achieve delivery, biocompatibility, and controlled drug release. However, the production of such exosomes typically requires stringent manufacturing conditions, resulting in low production yields and poor storage stability. Consequently, the development and industrial application of exosomes in the biomedical field have been significantly constrained. Moreover, conventional exosomes are generally utilized merely as passive carriers and do not possess direct therapeutic functionality. Therefore, there exists an urgent need in the art to develop an exosome that is readily manufacturable, exhibits high storage efficiency, and possesses intrinsic therapeutic efficacy.

### SUMMARY OF THE INVENTION

To develop an exosome that is easy to manufacture, exhibits high storage efficiency, and possesses intrinsic therapeutic efficacy, the present invention provides a corneal tissue exosome obtained by culturing a corneoscleral tissue, wherein steps for obtaining the corneal tissue exosome comprise collecting a corneoscleral tissue and disrupting the corneoscleral tissue, wherein the corneoscleral tissue comprises a ring-shaped limbal tissue; dispersing the disrupted corneoscleral tissue into multiple culture dishes and culturing in a culture environment, wherein the culture environment comprises a serum-free medium and a culture duration ranging from 12 to 72 hours, and wherein no collagenase is added to the corneoscleral tissue before or during culture; and collecting the serum-free medium in the culture dish that has been used to culture the corneoscleral tissue, centrifuging the serum-free medium to obtain a supernatant, and filtering the supernatant through a 0.22 µm membrane to obtain the corneal tissue exosome.

Wherein, the corneoscleral tissue is a tissue explant from a mammalian source.

Further, the corneoscleral tissue is a corneoscleral tissue remaining after corneal transplantation ex vivo, which is rich in limbal tissue. The corneoscleral tissue does not include a conjunctival tissue, an endothelium cell, or an iris tissue.

Wherein, the corneoscleral tissue include a scleral tissue, a corneal tissue, and a stromal cell.

Wherein, the corneal tissue exosome comprises expression of an integrin α9 protein and/or a CD59 protein.

The present invention further provides a method for manufacturing a corneal tissue exosome from a corneoscleral tissue. The method comprises steps of:
collecting the corneoscleral tissue and culturing the corneoscleral tissue and disrupting the corneoscleral tissue, wherein the corneoscleral tissue comprises a ring-shaped limbal tissue, dispersing the disrupted corneoscleral tissue into multiple culture dishes and culturing in a culture environment, wherein the culture environment comprises a serum-free medium and a culture duration ranging from 12 to 72 hours, and wherein no collagenase is added to the corneoscleral tissue before or during culture; and
collecting the serum-free medium in a culture dish that has been used to culture the corneoscleral tissue, centrifuging the serum-free medium to obtain a supernatant and/or purifying the serum-free medium to obtain a concentrate, and filtering the supernatant or the concentrate through a 0.22 µm membrane to obtain the corneal tissue exosome.

The present invention further provides a method for collecting the corneoscleral tissue, comprising steps of:
under sterile conditions, scraping off a conjunctival tissue, an endothelium cell, and an iris tissue of the corneoscleral tissue using a No. 15 surgical blunt knife.

Wherein, the corneoscleral tissue is cultured in the culture environment starting from 10 to 17 days after being ex vivo.

Wherein, the corneal tissue exosome can be used in applications for wound repair, cancer therapy, or treatment of immune-related diseases.

The method for obtaining the corneal tissue exosome provided by the present invention, and the corneal tissue exosome obtained thereby, exhibit the following advantages:
the corneal tissue exosome can be directly collected from the corneoscleral tissue remaining after corneal transplantation, thereby enabling the reutilization of biological resources and reducing the generation of biomedical waste;
the overall process for obtaining the corneal tissue exosome from the corneoscleral tissue is simple and eliminates the thawing, isolation, and cell culture procedures required in conventional cell culture processes, which significantly reduces time and labor costs; the total production yield of the corneal tissue exosome is high, demonstrating great potential for large-scale production; and
the corneal tissue exosome obtained from the corneoscleral tissue exhibits specific protein expression and possesses therapeutic potential in wound repair, ocular disease treatment, cancer therapy, and immunological disorder management.

The present invention further provides a use of the corneal tissue exosome for cell repair or treatment of complement activation-related immune diseases, or for targeting cancer cells expressing osteopontin.

The present invention further provides a composition comprising the corneal tissue exosome as provided above.

### BRIEF DESCRIPTION OF THE DRAWINGS

FIG. 1 is a flowchart of a preferred embodiment of the present invention.
FIG. 2 is an electron microscopy image according to a preferred embodiment of the present invention.
FIGS. 3A to 3B are comparison charts of size and concentration of the corneal tissue exosome according to a preferred embodiment of the present invention.
FIGS. 4A to 4B are comparison charts of cell source, size, and concentration of the corneal tissue exosome according to a preferred embodiment of the present invention.
FIG. 5 and FIG. 6 are charts showing protein expression of the corneal tissue exosome according to a preferred embodiment of the present invention.
FIGS. 7A to 7B are comparison charts of growth factor expression in Experiment 3 according to a preferred embodiment of the present invention.
FIGS. 8A to 8B are comparison charts of cell migration ability in Experiment 6 according to a preferred embodiment of the present invention.
FIG. 9 is a comparison chart of cell migration ability of the corneal tissue exosome after TFF purification according to a preferred embodiment of the present invention.
FIGS. 10A to 10B are comparison charts of protein expression in Experiment 2 according to a preferred embodiment of the present invention.
FIG. 11 is an analysis chart of the affinity reaction between the corneal tissue exosome and osteopontin according to a preferred embodiment of the present invention.

### DETAILED DESCRIPTION OF THE PREFERRED EMBODIMENTS

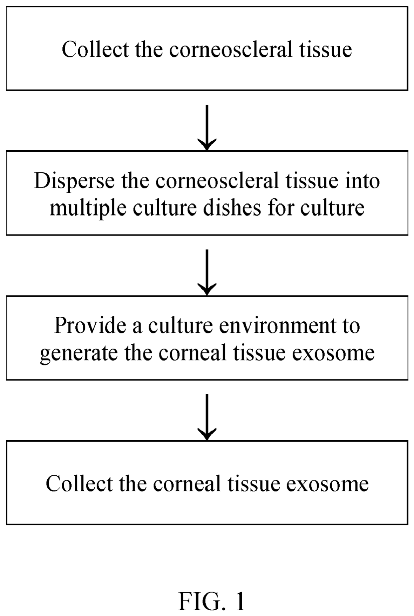

With reference to FIG. 1, the present invention provides a corneal tissue exosome exhibiting high expression of integrin α9, which is directly produced by culturing a corneoscleral tissue. The steps for collecting the corneal tissue exosome from the corneoscleral tissue are described as follows.

Step S1: Collecting the corneoscleral tissue.

The corneoscleral tissue is a tissue explant from a mammalian source.

The corneoscleral tissue is at least a portion of the transparent film-like cornea covering the forefront of an eyeball.

In this embodiment, the corneoscleral tissue is rich in limbal tissue. The limbal tissue is located at the outer periphery of the cornea and at the junction with the sclera.

Preferably, the corneoscleral tissue is a ring-shaped tissue remaining after corneal transplantation ex vivo.

Wherein, the corneoscleral tissue is cultured in the culture environment starting from 10 to 17 days after being ex vivo, so that the exosomes produced in accordance with the present invention possess consistency and homogeneity. With reference to Table 1, Samples 1 to 5 are groups where the corneoscleral tissue was cultured between 10 to 17 days after being ex vivo; Samples 6 to 9 are groups where the corneoscleral tissue was cultured 17 days or more after being ex vivo. The sources of the corneoscleral tissue for Samples 1 to 3 and 6 to 7 were Caucasian (U.S.), and the sources for Samples 4 to 5 and 8 to 9 were Asian (Taiwan). Through nanoparticle tracking analysis using ZetaView, it was found that exosomes collected from corneoscleral tissues of Samples 1 to 5 with an ex vivo time between 10 to 17 days exhibited a homogeneous particle size diameter (peak between 100-125 nm). For Samples 6 to 9, where the corneoscleral tissue had an ex vivo time of greater than or equal to 19 days, the collected exosomes showed high heterogeneity in particle size diameter (peak between 49.4-91.1 nm), with a tendency for the peak to shift to the left. Subsequent data will further demonstrate that exosomes generated from corneoscleral tissue cultured with an ex vivo time of 10 to 17 days not only exhibit high homogeneity but also show significant therapeutic efficacy in wound healing experiments, presenting dose-dependent therapeutic effects.

**Table 1**

| | Ex vivo time (days) | Particle size diameter peak (diameter/nm) |
|---|---|---|
| Sample 1 (U.S.) | 16 | 103.1 |
| Sample 2 (U.S.) | 16 | 106.1 |
| Sample 3 (U.S.) | 16 | 104.2 |
| Sample 4 (Taiwan) | 12 | 114.8 |
| Sample 5 (Taiwan) | 14 | 113.1 |

| | Ex vivo time (days) | Particle size diameter peak (diameter/nm) |
|---|---|---|
| Sample 6 (U.S.) | 20 | 49.4 |
| Sample 7 (U.S.) | 20 | 91.1 |
| Sample 8 (Taiwan) | 19 | 74.2 |
| Sample 9 (Taiwan) | 19 | 81.6 |

Preferably, the limbal tissue is a hollow ring shape, and a ratio of a minimum diameter constituted by its inner periphery to a maximum diameter constituted by its inner periphery is between 7:20 and 8.5:15.

Preferably, before culturing to generate the corneal tissue exosome, the corneoscleral tissue undergoes pretreatment to remove a conjunctival tissue, an endothelium cell, and an iris tissue.

Step S2: Dispersing the corneoscleral tissue into multiple culture dishes for culture.

The corneoscleral tissue may be dispersed into 2, 6, 15, or 24 culture dishes based on size or subsequent operational requirements, which is not limited in the present invention.

In a first embodiment, the corneoscleral tissue is cut into multiple corneal flaps, and the multiple corneal flaps are dispersed into multiple culture dishes. In this embodiment, the ring-shaped corneoscleral tissue is divided into six equal parts to form the six corneal flaps, and the six corneal flaps are respectively placed in the individual culture wells of a 6-well culture dish for culture.

In a second embodiment, the corneoscleral tissue is first divided into six equal parts to form the six corneal flaps. Each of the corneal flap is then cut into small fragments with scissors and placed in each culture well of the 6-well culture dish for culture; or alternatively, the six corneal flaps are first homogenized using a homogenizer to form a corneal homogenate, which is then placed in each culture well of the 6-well culture dish for culture.

In the second embodiment, the corneal homogenate is prepared by placing the corneoscleral tissue in the serum-free medium and disrupting it via a homogenizer. The homogenization is performed under temperature conditions of 2-8°C, frequency conditions of 50-80 Hz, and a duration of 100-140 seconds per cycle. The homogenization may be repeated several times as required, with each cycle separated by an interval of 30 seconds to 2 minutes, while maintaining the sample on ice.

Step S3: Providing a culture environment to generate the corneal tissue exosome.

The culture environment includes a serum-free medium (SFM) and a culture duration ranging from 12 to 72 hours.

The serum-free medium (SFM) may optionally comprise one or more of the following: MEM (Minimal Essential Medium), α-MEM (Alpha Minimal Essential Medium), DMEM (Dulbecco's Modified Minimal Essential Medium), IMDM (Iscove's Modified Dulbecco's Medium), MSCM (Mesenchymal Stem Cell Medium), SF-1 (SF1 hMSC Medium), MSC NutriStem^{®} XF Medium, CellCor^{™} EXO CD Medium, MesenCult^{™}-ACF Plus Culture Kit Medium, or HEK GM Medium.

Further, the appropriate amount of the serum-free medium is determined based on the type of culture dish and the size of the corneal flap in each dish. Preferably, 0.5 to 3 milliliters of the serum-free medium is added to each culture dish.

In this embodiment, the serum-free medium (SFM) is α-MEM (Alpha Minimal Essential Medium), and 1 milliliter of the serum-free medium is added to each culture dish, with a culture duration of 24 hours.

Step S4: Collecting the corneal tissue exosome.

The serum-free medium in the culture dish that has been used to culture the corneoscleral tissue is collected and subjected to a batch centrifugation process. A supernatant obtained is filtered through a 0.22 µm membrane to yield a filtered corneal tissue exosome, which can then be preserved.

Wherein, the corneal tissue exosome is stored in a frozen environment at -80°C for at least four months.

Wherein, the batch centrifugation sequentially includes centrifugation at 200-500 g for 10-20 minutes, followed by centrifugation at 1,000-2,000 g for 15-30 minutes, and centrifugation at 9,000-14,000 g for 30-60 minutes.

In one embodiment, the serum-free medium undergoes the batch centrifugation process, and the resulting supernatant is filtered to obtain the corneal tissue exosome. The production yield of the corneal tissue exosome can reach 1.1 × 10¹¹ to 5.2 × 10¹¹ particles per milliliter. Upon conversion, the total yield from one corneoscleral tissue can stably range from approximately 1.8 × 10¹² to 6.7 × 10¹² exosomes within 24 hours.

Step S5 (optional): Purifying the corneal tissue exosome.

After the supernatant is filtered through the 0.22 µm membrane and redissolved, tangential flow filtration (TFF) is applied for further purification and concentration of the corneal tissue exosome. Through appropriate selection of osmotic pressure and filtration membrane parameters, the TFF process can remove approximately 90% of impurities from the corneal tissue exosome preparation.

In addition, in other embodiments, the method for collecting the corneal tissue exosome from the corneoscleral tissue as described above may be combined with other conventional techniques for exosome separation, purification, or extraction, including, but not limited to, size-exclusion chromatography (SEC), ultrafiltration (UF), precipitation, or immunoaffinity (IA) methods.

### Experiment 1

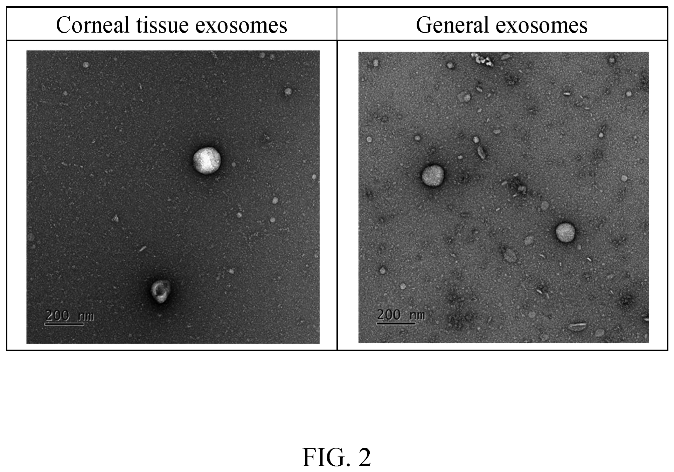

The present invention further analyzes the exosomes produced by the method provided above. The exosomes produced from the corneoscleral tissue used in Experiment 1 were all obtained via the first embodiment described above. As shown in the results of FIG. 2, there is no significant difference in morphology or size between the corneal tissue exosome produced by directly culturing the corneoscleral tissue and general exosomes obtained from mesenchymal stem cells (MSCs). Similarly, nanoparticle tracking analysis using the ZetaView system reveals that the diameter peak of the exosome produced by the present method ranges from 75 to 125 nm, indicating that the corneal tissue exosome conforms to the general exosome standard diameter range of 30 to 200 nm.

Further analyses were conducted to determine whether corneal tissue exosomes derived from corneoscleral tissues under different conditions exhibit variations.

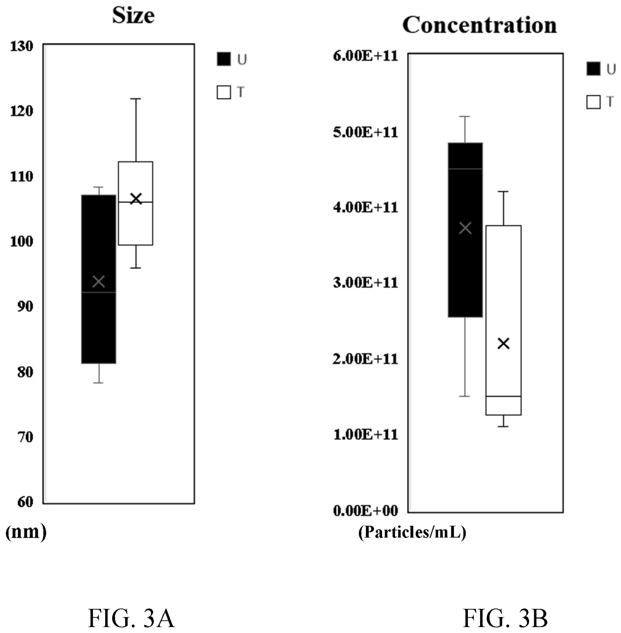

FIGS. 3A to 3B show the corneal tissue exosomes obtained by culturing corneoscleral tissues from a Caucasian donor (U.S., U) and an Asian donor (Taiwan, T), respectively, according to the first embodiment. The results indicate that in both the Caucasian donor (U.S., U) group and the Asian donor (Taiwan, T) group, the corneal tissue exosomes exhibit no significant difference in particle size or concentration, demonstrating that the corneal tissue exosomes obtained by the present invention possess high homogeneity.

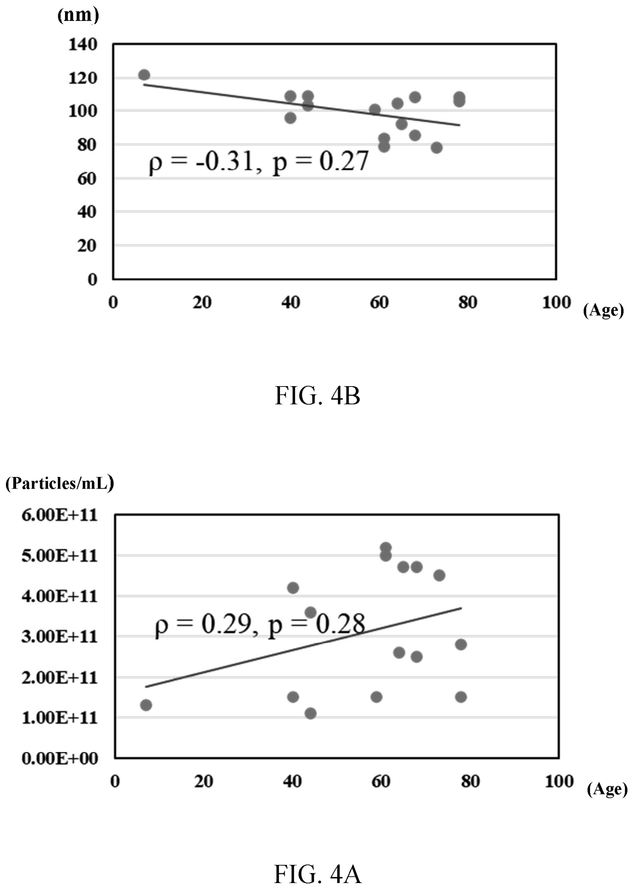

FIGS. 4A to 4B compare corneal tissue exosomes cultured from corneoscleral tissues of donors of different ages. The results demonstrate that donor age does not affect either the particle size or the production concentration of the corneal tissue exosome, nor does it influence the preservation stability of the corneal tissue exosome, indicating high homogeneity of the corneal tissue exosomes obtained by the present invention.

Further comparisons were made between the corneal tissue exosomes (U1-U9, T1-T4, T8, and T10) obtained using the method in accordance with the present invention and conventional exosomes obtained from mesenchymal stem cells (MSCs) or retinal pigment epithelial (RPE) cells.

With reference to Table 2, nanoparticle tracking analysis using ZetaView indicates that the production concentration of the corneal tissue exosome produced by the present method ranges between 1.1 × 10¹¹ and 5.2 × 10¹¹ particles per milliliter, indicating that the yield per unit time of the corneal tissue exosome obtained by the present invention is higher than that of conventional cell culture methods.

It is noteworthy that the method for obtaining the corneal tissue exosome provided by the present invention is characterized by procedural simplicity and low time cost. From tissue collection to product acquisition, only 24 hours of rapid culture are required to stably produce up to 1.8 × 10¹² to 6.7 × 10¹² corneal tissue exosomes. In contrast, obtaining general exosomes through conventional culture methods typically requires cell lines or isolation of target cells from tissue for culture. It often takes more than two weeks or even several months to reach conditions suitable for harvesting general exosomes, and the quantity of general exosomes obtained cannot match the quantity achieved by the present application with only 24 hours of culture. Additionally, the corneal tissue exosome obtained using the method of the present invention exhibits long-term storage stability, thereby reducing the frequency with which operators need to repeat the exosome culture procedures. This feature provides significant potential to accelerate scientific research and pharmaceutical development in the biomedical industry.

Table 2 compares Mesenchymal Stem Cells (MSC) or Retinal Pigment Epithelial (RPE) cell line ARPE19 in a 175T culture flask. Even when cultured continuously for 72 hours, the yield fails to reach the production yield achieved by the corneoscleral tissue cultured for only 24 hours according to the present invention. This finding confirms that the method for obtaining the corneal tissue exosome provided by the present invention possesses a strong potential for mass production.

**Table 2**

| | Corneal Tissue Exosome (First Embodiment) | General Exosome (1) | General Exosome (2) |
|---|---|---|---|
| Source | The corneoscleral tissue | Mesenchymal stem cells | Retinal pigment epithelial cell line (ARPE19) |
| Diameter Peak Value (diameter/nm) | 75-125 | 131 | 112 |
| Production Concentration (particles/ml) | 1.1-5.2 × 10¹¹ | 1.4 × 10¹¹ | 1.3 × 10¹¹ |
| Total Production Yield | 1.8-6.7 × 10¹² (24 hours) | 7 × 10¹¹ (72 hours) | 7 × 10¹¹ (72 hours) |

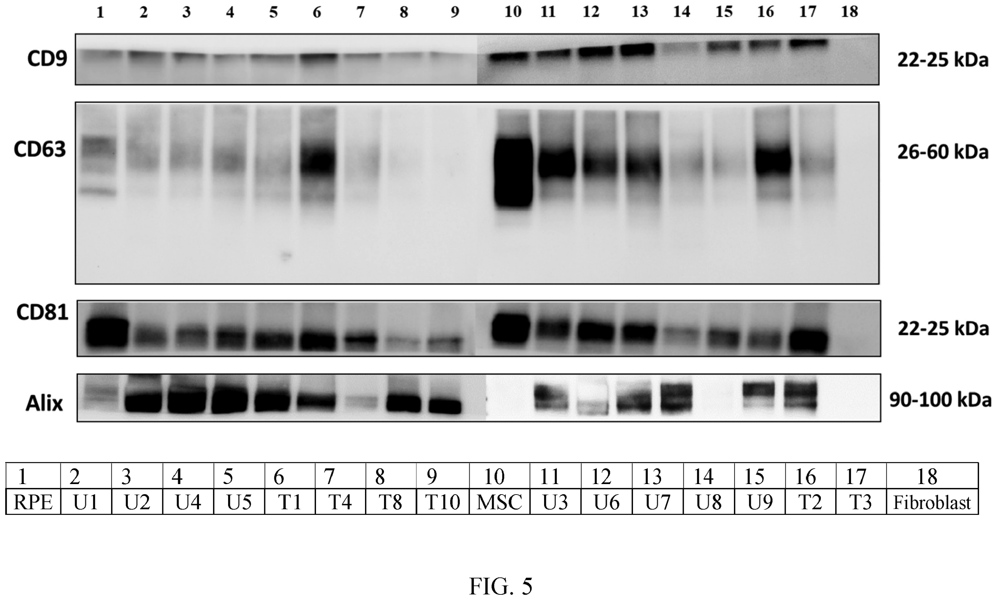

Further, the present invention compares the expression levels of common marker proteins found in general exosomes. As shown in FIG. 5, western blot analysis reveals that the corneal tissue exosome exhibits expression of CD63, CD9, CD81, and Alix proteins. Wherein, sample 18 is fibroblast cell which is applied as a negative control. These findings indicate that the corneal tissue exosome obtained by the method of the present invention possesses strong potential for genetic engineering modification.

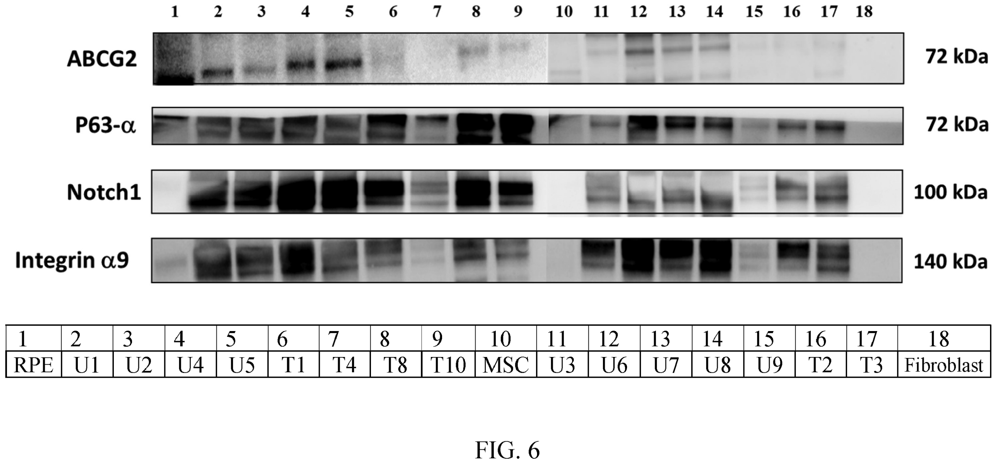

The present invention also evaluates the expression of marker proteins common to limbal stem cells in the limbal tissue, P63-α and integrin α9, as well as marker proteins common to general stem cells, ABCG2 and Notch1, in the corneal tissue exosome. Using western blot analysis, as shown in FIG. 6, the corneal tissue exosomes (U1-U9, T1-T4, T8, and T10) obtained using the method of the present invention exhibit high expression of Notch1 protein and integrin α protein. Wherein, sample 18 is fibroblast cell which is applied as a negative control. These results demonstrate that the corneal tissue exosome obtained by the method of the present invention reflects the characteristics of the limbal tissue and limbal stem cells.

### Experiment 2

The present invention further compares the corneal tissue exosome obtained by the method of the present invention with exosomes obtained from other ocular tissues or cells. The corneal tissue exosomes used in Experiment 2 were all obtained via the second embodiment described above. The groups in Experiment 2 include:
Experimental Group 1: The corneal tissue exosome produced from the corneoscleral tissue via the second embodiment described above;
Experimental Group 2: A central corneal tissue exosome produced via the second embodiment described above from a central cornea of a cornea (i.e., the corneal tissue with the limbal tissue removed);
Experimental Group 3: The corneal tissue exosome produced via the second embodiment described above from the corneoscleral tissue removed from the cornea in Experimental Group 2; and
Control Group 1: General exosomes obtained from the culture medium of a Retinal Pigment Epithelial cell line ARPE19 after culture.

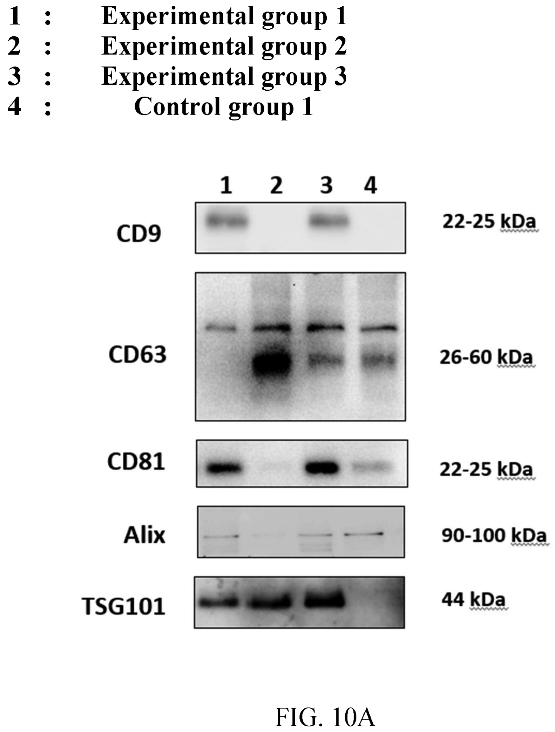

With reference to FIG. 10A, in the western blot results, Experimental Groups 1 to 3 (the obtained corneal tissue exosome and central corneal tissue exosome) and Control Group 1 (general exosome) all show a certain level of expression of common exosome marker proteins. Notably, compared to other groups, Experimental Groups 1 and 3, which utilized the corneoscleral tissue rich in limbal tissue, produced corneoscleral tissue exosomes with high expression of CD9 and CD81 proteins.

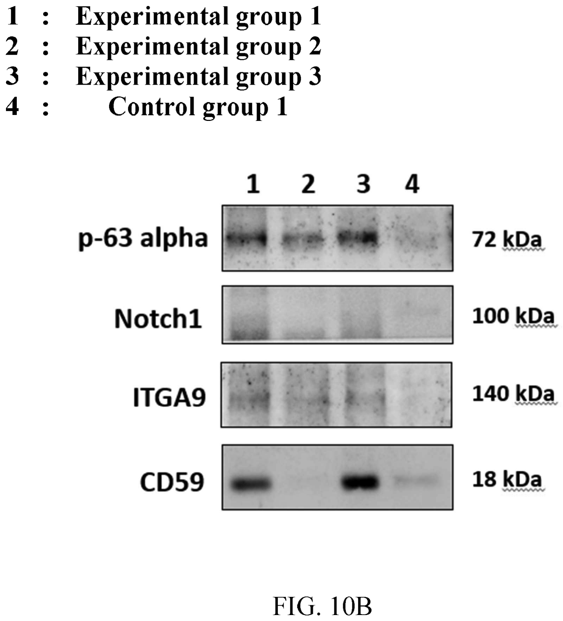

Next, with reference to FIG. 10B, Experimental Groups 1 to 3 all exhibit expression of P63-α, Notch1, and Integrin α9 proteins (with expression being most significant in the corneal tissue exosomes obtained in Experimental Groups 1 and 3), whereas the exosomes obtained in Control Group 1 show almost no expression of these proteins. Notably, compared to other groups, Experimental Groups 1 and 3 highly express CD59 protein via the corneal tissue exosome. This highlights the uniqueness of the corneal tissue exosome secreted by the corneoscleral tissue; its CD59-rich characteristic makes it a preferred candidate platform for drug delivery applications.

### Experiment 3

Experiments 1 and 2, along with FIGS. 6, 10A, and 10B, confirmed that the corneal tissue exosome is rich in Integrin α9 expression. According to a previous report (doi: 10.3390/cancers12113379), Integrin α9 can be regarded as a receptor for the SVVYGLR domain. The SVVYGLR domain possesses specificity and is commonly found in osteopontin (OPN), which is highly and excessively expressed in various cancer cells, including but not limited to breast cancer, prostate cancer, colorectal cancer, head and neck cancer, liver cancer, and lung cancer. The present invention further tests whether the Integrin α9-bearing corneal tissue exosome (Integrin α9⁺ EV) can produce a targeting effect on osteopontin. The groups in Experiment 3 include:
Experimental Group 1: The corneal tissue exosome produced from the corneoscleral tissue via the second embodiment described above, dissolved in 1 mL of Dulbecco's Phosphate Buffered Saline (DPBS);
Control Group 1: General exosomes obtained from the culture medium of a Retinal Pigment Epithelial cell line ARPE19 after culture, dissolved in 1 mL of Dulbecco's Phosphate Buffered Saline (DPBS); and
Control Group 2: Phosphate Buffered Saline (PBS).

Experimental Group 1 and Control Group 1 were analyzed via Nanoparticle Tracking Analysis (NTA) to determine the exosome content in each group. The concentration for Experimental Group 1 was 1.6 × 10¹¹ particles/mL, and for Control Group 1 was 1.1 × 10¹¹ particles/mL.

The affinity of the above groups for osteopontin was further tested via fluorescent staining. The overall process was as follows:
1. Add 100 µl of coating buffer containing osteopontin to each well of a 96-well plate, wherein the content of osteopontin is 200 ng, and allow to react statically at 4°C for 8-12 hours;
2. Take 100 µl from Experimental Group 1 and Control Group 1 respectively, mix uniformly with 1-5 µl of fluorescent dye (PKH67), then sequentially add 1 mL of Diluent C and 200 µl of 10% BSA dissolved in PBS, mix uniformly, and incubate at room temperature for 5-40 minutes;
4. Perform ultracentrifugation (100,000 × g) for 1 hour to collect the fluorescently labeled corneal tissue exosomes and general exosomes from the sediment, then redissolve in 100 µl of PBS;
5. Add each group to the wells of the reaction plate with the added coating solution, and react at room temperature for 20-30 minutes;
6. After the reaction, discard the solution, wash with PBS, and observe the fluorescent reaction under a fluorescence microscope.

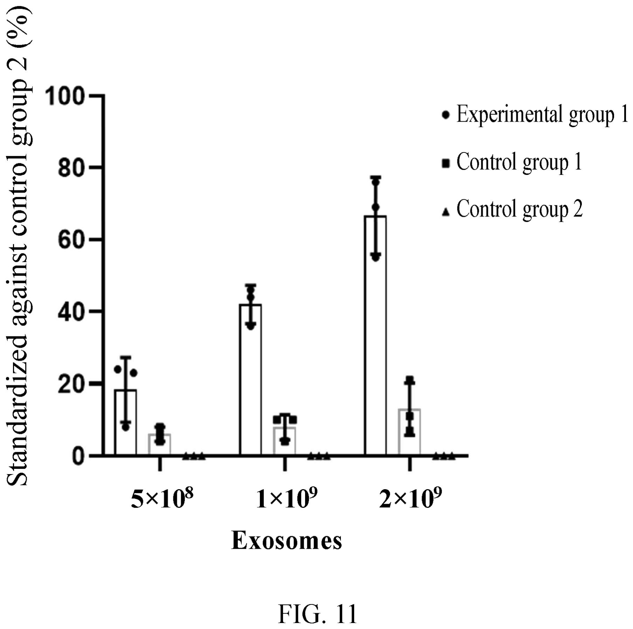

Referring to FIG. 11, Experimental Group 1 and Control Group 1 performed the osteopontin affinity reaction with exosome contents of 5 × 10⁸ / µl, 1 × 10⁹ / µl, and 2 × 10⁹ / µl, respectively. The results show that the corneal tissue exosome provided by Experimental Group 1 exhibits a superior affinity reaction compared to the general exosome provided by Control Group 1. Furthermore, as the exosome content increases, the binding effect of Experimental Group 1 with osteopontin significantly improves.

The corneal tissue exosome (Integrin α9⁺ EV) is capable of binding to cancer cells (such as breast cancer, prostate cancer, colorectal cancer, head and neck cancer, liver cancer, and lung cancer) that overexpress osteopontin (OPN). This enhances the specificity of tumor targeting toward the SVVYGLR domain, avoiding the nonspecific tissue toxicity commonly associated with binding to the RGD domain on OPN. Preliminary validation shows that the corneal tissue exosome (Integrin α9⁺ EV) has a higher degree of binding to OPN protein compared to general exosomes with low expression of Integrin α9 protein, indicating that the corneal tissue exosome (Integrin α9⁺ EV) serves as a promising candidate platform for drug delivery in future cancer therapy.

In addition, when the corneal tissue exosome exhibits high CD59 expression, the corneal tissue exosome (CD59⁺ EV) can inhibit the formation of a membrane attack complex (MAC) assembly following complement activation in immune responses. Thus, the corneal tissue exosome (CD59⁺ EV) provides a potential platform for drug delivery in the treatment of complement activation disorders, inflammatory diseases, and autoimmune diseases.

Based on the foregoing, corneal tissue exosomes expressing specific proteins (such as Integrin α9⁺ EV or CD59⁺ EV) can be selectively isolated according to therapeutic needs to achieve targeted drug or therapeutic delivery toward specific diseases, cells, or tissues. Selection of an exosome with a specific protein expression profile can be achieved using tangential flow filtration (TFF) technology. During purification, affinity chromatography can be applied in parallel, employing antibodies such as a CD59 antibody or an integrin α9 antibody to classify the exosomes based on whether they express the specific protein.

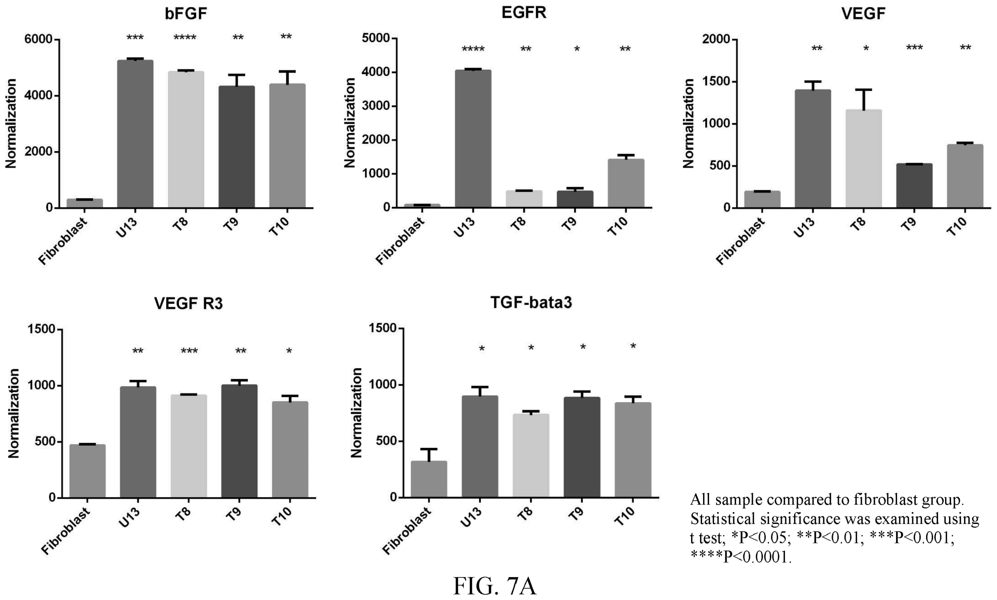

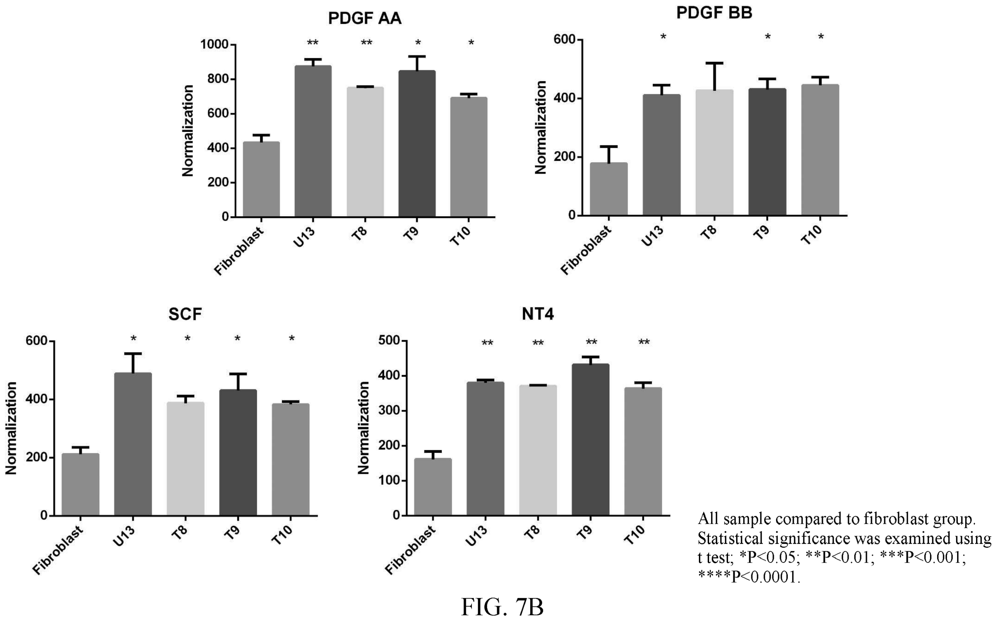

Further referring to FIGS. 7A and 7B, when analyzing the expression levels of multiple growth factors in the corneal tissue exosome and general exosomes, it is observed that the corneal tissue exosome obtained using the method of the present invention is enriched in a variety of growth factors, particularly bFGF, EGFR, VEGF, VEGFR3, TGF-β3, PDGF-AA, PDGF-BB, SCF, NT4, and IGFBP1. These findings highlight the potential of the corneal tissue exosome in cell or tissue repair.

### Experiment 4

Furthermore, the present invention compares the production concentrations of the corneal tissue exosome in the first embodiment and the second embodiment, and simultaneously examines whether differences in ethnicity, tissue disruption methods, and filtration through the 0.22 µm membrane affect the production concentration of the corneal tissue exosome (as shown in Tables 3 to 5).

As shown in Table 3 corresponding to the first embodiment, the supernatant derived from corneoscleral tissues of donors of different ethnic backgrounds does not affect the final particle size or production concentration of the corneal tissue exosome. This demonstrates that the corneal tissue exosome obtained by the method of the present invention possesses excellent homogeneity, resists aggregation and precipitation, and exhibits batch-to-batch stability. Consistent results are obtained in Tables 3 and 4 below.

The results in Table 4 compare the second embodiment. The production concentration of the corneal tissue exosome obtained by cutting the corneoscleral tissue into small fragments with scissors or disrupting it with a homogenizer can be increased by approximately one order of magnitude compared to the first embodiment. Additionally, filtration through the 0.22 µm membrane does not affect the production concentration of the corneal tissue exosome.

**Table 3, First Embodiment**

| Tissue Dispersion Method | Cut into six corneal flaps | |
|---|---|---|
| Race | Caucasian (US) | Asian (Taiwan) |
| Membrane Filtration | V | V |
| Particle Size (diameter/nm) | 104.2 | 101.5 |
| Production Concentration (particles/ml) | 2.6 × 10¹¹ | 2.9 × 10¹¹ |

**Table 4, Second Embodiment**

| Tissue Dispersion Method | Cut into small fragments with scissors | Homogenizer disruption | |
|---|---|---|---|
| Membrane Filtration | V | V | X |
| Production Concentration (particles/ml) | 1.0 × 10¹² | 1.3 × 10¹² | 1.6 × 10¹² |

### Experiment 5

Further comparison is made between the technology provided by the present invention and current technology involving culturing ex vivo tissue using collagenase. Wherein, the groups in Experiment 5 include:
Experimental Group 1: The corneoscleral tissue was cut into small fragments with scissors and then cultured using the method provided in the second embodiment of the present invention.
Comparative Example 1: After the corneoscleral tissue was cut into small fragments with scissors, collagenase was added and mixed for a reaction of 30 minutes to decompose the fragmented corneoscleral tissue.
Comparative Example 2: After the corneoscleral tissue was cut into small fragments with scissors, collagenase was added and mixed for a reaction of 60 minutes to decompose the fragmented corneoscleral tissue.
In Comparative Examples 1 and 2, after the corneoscleral tissue reacted with collagenase, the collagenase was removed by washing with a buffer solution (such as Phosphate Buffered Saline, PBS), and then the corneoscleral tissue was placed in a culture well for culture.

The results in Table 5 reveal that Experimental Group 1, without the addition of collagenase, produced a yield of corneal tissue exosomes significantly superior to Comparative Examples 1 and 2 where collagenase was added. A further investigation into the reason suggests that the stiffness of the corneoscleral tissue is higher compared to general tissues, lying between the cornea and the sclera, with a Young's modulus of about 100-5000 kpa (retina is about 0.1-10 kpa; visceral tissue is about 0.5-30 kpa). Consequently, the corneoscleral tissue is not only difficult to decompose via the collagenase reaction, but the reaction may even lead to a reduction in the production efficiency of the corneal tissue exosome.

Summarizing the results from comparisons in Tables 2 to 4, it can be seen that in the second embodiment, the corneal tissue flap yields more corneal tissue exosomes after homogenization under moderate conditions and culture, which is approximately four times the yield in the first embodiment. Furthermore, the present invention obviates the need for collagenase treatment, marking a significant breakthrough from prior art concepts and demonstrating the mass production potential of increased yield following process optimization.

**Table 5**

| Group | Experimental Group 1 | | Comparative Example 1 | | Comparative Example 2 | |
|---|---|---|---|---|---|---|
| Membrane Filtration | X | V | X | V | X | V |
| Production Concentration (particles/ml) | 7.3 × 10¹¹ | 6.5 × 10¹¹ | 4.5 × 10¹¹ | 4.4 × 10¹¹ | 4.5 × 10¹¹ | 4.9 × 10¹¹ |

### Experiment 6

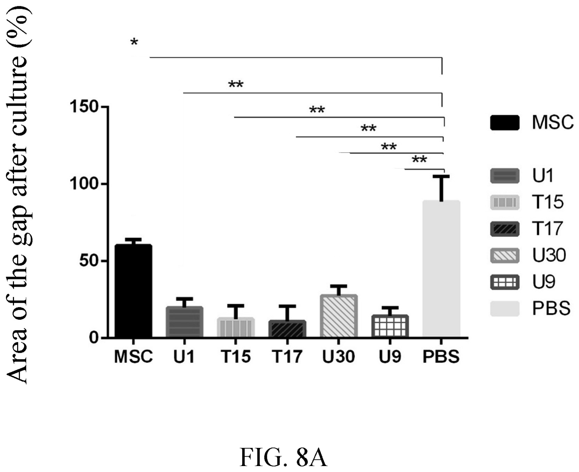

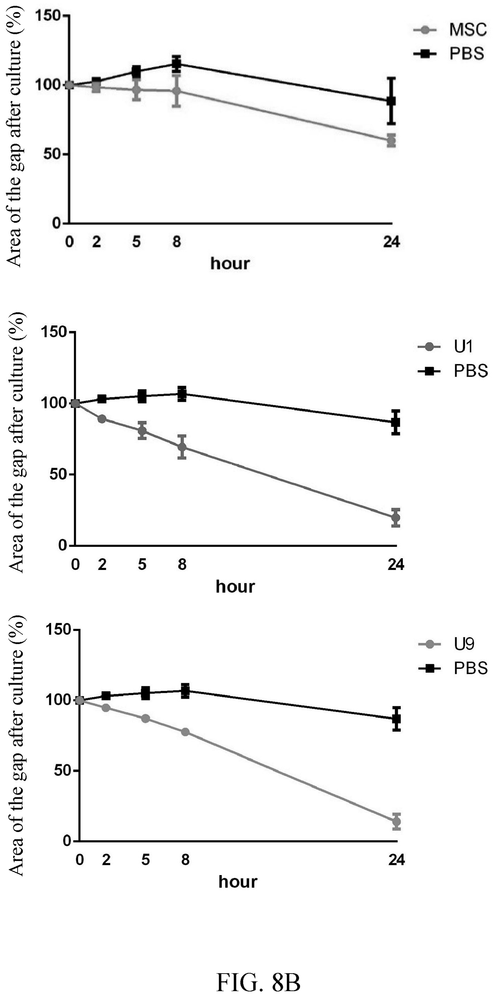

The cellular repair ability of the corneal exosome provided by the present invention was further analyzed. Human corneal epithelial cells (HCE-T) were cultured on a cell plate to conduct a wound healing assay (scratch assay). A gap (wounding area) was scraped in the confluent HCE-T cell layer, and the following treatments were administered respectively: a PBS control group, general exosomes (2 × 10⁸ particles) derived from mesenchymal stem cells, and the corneal tissue exosome (2 × 10⁸ particles) obtained by the method of the present invention (randomly selected U1, U9, U30, T15, and T17), to evaluate the effect of each group on cell migration ability. It can be found that after 24 hours (FIG. 8A), in the groups treated with the corneal tissue exosome obtained by the method of the present invention, the wounding area in the cell plate was significantly reduced. This confirms that the corneal tissue exosome obtained by the method of the present invention has similar therapeutic efficacy for wound repair and is better than the conventionally used general exosomes derived from mesenchymal stem cells. The difference is even more apparent in the chart analyzing the area of the wounding area for each group at 0 hour, 2 hours, 5 hours, 8 hours, and 24 hours after treatment (FIG. 8B).

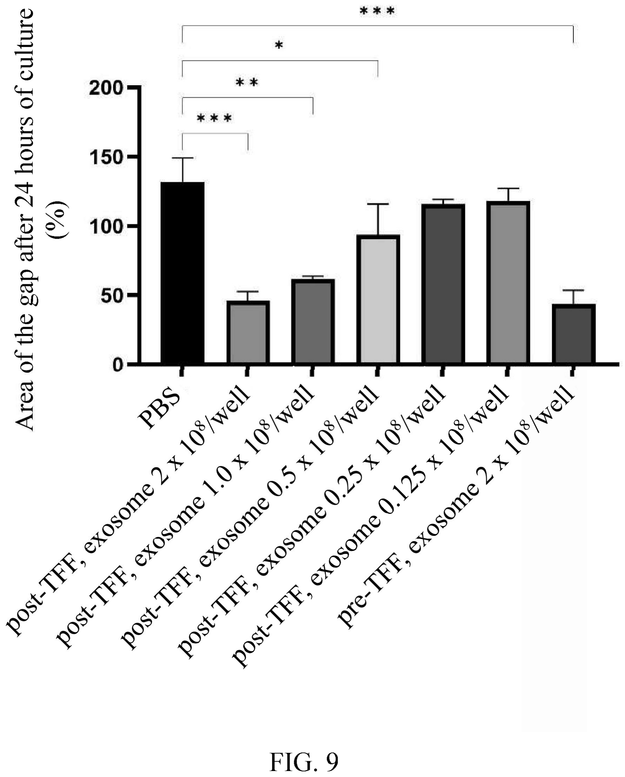

FIG. 9 compares whether corneal tissue exosomes purified via TFF in Step S5 affect their cellular repair ability. A wound healing assay (scratch assay) was similarly conducted, and the following groups were administered: a PBS control group; an unpurified corneal tissue exosome (pre-TFF) group at a concentration of 2 × 10⁸; and TFF-purified corneal tissue exosome (post-TFF) groups at concentrations of 2 × 10⁸, 1.0 × 10⁸, 0.5 × 10⁸, 0.25 × 10⁸, and 0.125 × 10⁸, to evaluate the effect of each group on cell migration ability.

The results show that the TFF-purified corneal tissue exosome (post-TFF) exhibits the same potency for cell repair as the unpurified corneal tissue exosome (pre-TFF). Moreover, the TFF-purified corneal tissue exosome (post-TFF) demonstrates a dose-dependent effect between 2 × 10⁸ and 0.125 × 10⁸. Even at a concentration of 0.125 × 10⁸, the effect on HCE-T cell migration ability within 24 hours remains significantly different, unaffected by the TFF purification process. This indicates that the capability of the corneal tissue exosome to repair corneal wounds allows it to be developed as a candidate drug for treating ocular diseases, including but not limited to dry-eye syndrome, corneal erosion, corneal ulceration, etc.

The method for obtaining the corneal tissue exosome provided by the present invention, and the corneal tissue exosome obtained thereby, offer the following advantages:
The corneal tissue exosome can be rapidly collected directly from the corneoscleral tissue after corneal transplantation, enabling reutilization of biological resources and reducing the generation of biomedical waste.
The overall process for obtaining the corneal tissue exosome from the corneoscleral tissue is simple and eliminates the thawing, isolation, and cell culture procedures required in conventional cell culture processes. This significantly reduces time and labor costs, and the total production yield of the corneal tissue exosome is high, demonstrating great potential for large-scale production.
The corneal tissue exosome obtained from the corneoscleral tissue exhibits specific protein expression and possesses therapeutic potential for wound repair, ocular disease treatment, cancer therapy, and immune-related disease management.

## Claims

1. A corneal tissue exosome obtained by culturing a corneoscleral tissue, wherein steps for obtaining the corneal tissue exosome comprise:
collecting a corneoscleral tissue and disrupting the corneoscleral tissue, wherein the corneoscleral tissue comprises a ring-shaped limbal tissue;
dispersing the disrupted corneoscleral tissue into multiple culture dishes and culturing in a culture environment, wherein the culture environment comprises a serum-free medium and a culture duration ranging from 12 to 72 hours, and wherein no collagenase is added to the corneoscleral tissue before or during culture; and
collecting the serum-free medium in the culture dish that has been used to culture the corneoscleral tissue, centrifuging the serum-free medium to obtain a supernatant, and filtering the supernatant through a 0.22 µm membrane to obtain the corneal tissue exosome.

2. The corneal tissue exosome according to claim 1, wherein the corneoscleral tissue is a tissue explant from a mammalian source, and wherein culturing in the culture environment is initiated after 10 to 17 days ex vivo.

3. The corneal tissue exosome according to claim 2, comprising the limbal tissue, wherein a ratio of a minimum diameter constituted by an inner periphery of the limbal tissue to a maximum diameter constituted by the inner periphery is between 7:20 and 8.5:15.

4. The corneal tissue exosome according to claim 3, wherein the corneoscleral tissue does not include a conjunctival tissue, an endothelium cell, or an iris tissue.

5. The corneal tissue exosome according to claim 1, comprising expression of an integrin α9 protein.

6. The corneal tissue exosome according to claim 1, comprising expression of a CD59 protein.

7. A method for manufacturing a corneal tissue exosome from a corneoscleral tissue, comprising steps of:
collecting a corneoscleral tissue and disrupting the corneoscleral tissue, wherein the corneoscleral tissue comprises a ring-shaped limbal tissue;
dispersing the disrupted corneoscleral tissue into multiple culture dishes and culturing in a culture environment, wherein the culture environment comprises a serum-free medium and a culture duration ranging from 12 to 72 hours, and wherein no collagenase is added to the corneoscleral tissue before or during culture; and
collecting the serum-free medium in the culture dish that has been used to culture the corneoscleral tissue, centrifuging the serum-free medium to obtain a supernatant, and filtering the supernatant through a 0.22 µm membrane to obtain the corneal tissue exosome.

8. The method according to claim 7, wherein the corneoscleral tissue is a tissue explant from a mammalian source, and wherein culturing in the culture environment is initiated after 10 to 17 days ex vivo.

9. The method according to claim 8, wherein a ratio of a minimum diameter constituted by an inner periphery of the limbal tissue to a maximum diameter constituted by the inner periphery is between 7:20 and 8.5:15.

10. The method according to claim 9, wherein the corneoscleral tissue is fragmented or homogenized and then dispersed into the multiple culture dishes for culture.

11. The method according to claim 10, wherein the serum-free medium (SFM) comprises a MEM (Minimal Essential Medium), an α-MEM (Alpha Minimal Essential Medium), or a DMEM (Dulbecco's Modified Minimal Essential Medium).

12. The method according to claim 7, wherein the centrifuging sequentially comprises centrifuging at 200-500 g for 10-20 minutes, centrifuging at 1,000-2,000 g for 15-30 minutes, and centrifuging at 9,000-14,000 g for 30-60 minutes.

13. The method according to claim 12, wherein the supernatant is filtered through a 0.22 µm membrane to obtain the corneal tissue exosome.

14. The method according to claim 13, wherein the supernatant is purified and subsequently filtered through a 0.22 µm membrane to obtain the corneal tissue exosome, wherein the purification comprises tangential flow filtration (TFF), size-exclusion chromatography (SEC), ultrafiltration (UF), precipitation, or immunoaffinity (IA).

15. A use of the corneal tissue exosome according to claim 1 for cell repair or treatment of complement activation-related immune diseases.

16. A use of the corneal tissue exosome according to claim 5 for targeting cancer cells expressing osteopontin.

17. A composition comprising the corneal tissue exosome according to claim 1.
